# EUROPEAN PATENT APPLICATION

(11) **EP 3 454 057 A1**
(43) Date of publication of application: **13.03.2019**
(21) Application number: 18189520.2
(22) Date of filing: 17.08.2018
(51) Int. Cl.: G01N 27/327, C12Q 1/00, G01N 33/72

(54) **MEASURING METHOD AND MEASURING APPARATUS**

(30) Priority: 17.08.2017 JP 2017157636; 09.08.2018 JP 2018150825
(71) Applicant: ARKRAY, Inc., Kyoto-shi, Kyoto 601-8045 (JP)
(72) Inventor: KANEDA, Hisashi, Kyoto-shi, Kyoto 602-0008 (JP); NAKATANI, Ayano, Kyoto-shi, Kyoto 602-0008 (JP)
(74) Representative: Dehns

(57) **Abstract**

A measuring method includes applying a first signal having a first value to a sample disposed in the flow passage by using a working electrode (5) and a counter electrode (4), measuring a first electric response value of the sample to the first signal, applying continuously for a given time a second signal having a second value higher than the first value to the sample disposed in the flow passage by using the working electrode (5) and the counter electrode (4), measuring a second electric response value of the sample within the given time to the second signal, the second electric response value indicating amount of electric charge generated by electrolysis of water in the sample, and correcting a value obtained from the first electric response value based on the second electric response value.

## Description

### FIELD

The present disclosure relates to a measuring method and a measuring apparatus.

### BACKGROUND

Conventionally, a technique is known, in which a a value of concentration of glucose (glucose value) and a hematocrit value (Hct value) in blood, which is an example of the sample, are measured, and the glucose value is corrected by using the Hct value. For example, a method is known as the method for measuring the glucose value and the Hct value, in which the Hct value is measured by using a counter electrode and a working electrode provided with a mediator and the glucose value is measured by using a counter electrode and a working electrode provided with a reagent.

Alternatively, a method is known, in which the Hct value is measured by applying an alternative current (AC) voltage to a working electrode and a counter electrode provided with a reagent respectively, and the glucose value is measured by applying a direct current (DC) voltage. Further, a method is known, in which a common working electrode is used for the measurement of the glucose value and the measurement of the Hct value, and the both measurements are performed by applying a direct current voltage. Further alternatively, the following method is known. That is, a first electric response is measured with respect to a first signal inputted into a first electrode pair capable of making contact with a sample. A second signal, which is inputted into a second electrode pair capable of making contact with the sample, is used, the second signal having a value which changes from a first level to a second level, and the second signal thereafter maintaining the second level. A second electric response with respect to the second signal is measured as a peak value of a response signal with respect to the change of the second signal. A value, which indicates the amount of a target substance of the sample as obtained from the first electric response, is corrected based on the peak value of the response signal.

For further information, see Japanese Patent No. 4611208, Japanese Patent Application Laid-Open No. 2016-510124 (PCT), Japanese Patent Application Laid-Open No. 2005-147990, and Japanese Patent Application Laid-Open No. 2014-232102.

At least preferred embodiments of the invention provide a measuring method and a measuring apparatus which make it possible to accurately measure a target substance contained in a sample by using a new technique.

### SUMMARY

A first aspect of the invention provides a measuring method for measuring a target substance contained in a sample by using an analysis tool in which at least one working electrode provided with a reagent including an enzyme and at least one counter electrode are arranged in a flow passage, the measuring method including:
applying a first signal, which is a direct current signal, having a first value to the sample disposed in the flow passage by using the working electrode and the counter electrode;
measuring a first electric response value of the sample to the first signal;
applying continuously for a given time a second signal, which is a direct current signal, having a second value higher than the first value to the sample disposed in the flow passage by using the working electrode and the counter electrode;
measuring a second electric response value of the sample within the given time to the second signal, the second electric response value indicating amount of electric charge generated by electrolysis of water in the sample; and
correcting a value obtained from the first electric response value based on the second electric response value.

A second aspect of the invention provides a measuring apparatus for measuring a target substance contained in a sample by using an analysis tool in which at least one working electrode provided with a reagent including an enzyme and at least one counter electrode are arranged in a flow passage, the measuring apparatus including:
a first measuring unit configured to apply a first signal, which is a direct current signal, having a first value to the sample disposed in the flow passage by using the working electrode and the counter electrode, and configured to measure a first electric response value of the sample to the first signal;
a second measuring unit configured to apply continuously for a given time a second signal, which is a direct current signal, having a second value higher than the first value to the sample disposed in the flow passage by using the working electrode and the counter electrode, and configured to measure a second electric response value of the sample within the given time to the second signal, the second electric response value indicating amount of electric charge generated by electrolysis of water in the sample; and
a correcting unit configured to correct a value obtained from the first electric response value based on the second electric response value.

The advantages of at least preferred embodiments of the invention will be realized and attained by means of the elements and combinations particularly pointed out in the claims.

It is to be understood that both the foregoing general description and the following detailed description are exemplary and explanatory and are not restrictive of the invention.

### BRIEF DESCRIPTION OF THE DRAWINGS

Certain preferred embodiments of the invention will now be described by way of example only and with reference to the accompanying drawings in which:
FIG. 1A illustrates a top view illustrating an analysis tool (biosensor having two electrodes) according to an embodiment, and FIG. 1B illustrates a side view illustrating the biosensor illustrated in FIG. 1A;
FIG. 2A illustrates a top view illustrating an analysis tool (biosensor having three electrodes) according to an embodiment, and FIG. 2B illustrates a side view illustrating the biosensor illustrated in FIG. 2A;
FIG. 3 illustrates an exemplary arrangement of a measuring apparatus according to an embodiment;
FIG. 4 illustrates a flow chart illustrating an exemplary measuring method based on the use of the biosensor and the measuring apparatus according to the embodiment;
FIGS. 5A to 5F illustrate results of measurement of the Hct value and the glucose value according to Example 1;
FIGS. 6A to 6C illustrate the Hct linearity according to Example 1;
FIGS. 7A to 7F illustrate results of measurement of the Hct value and the glucose value according to Example 2;
FIGS. 8A to 8C illustrate the Hct linearity according to Example 2;
FIGS. 9A to 9E illustrate the Hct linearity and the time courses of respective applied voltages according to Example 3;
FIGS. 10A to 10E illustrate the Hct linearity and the time courses of respective applied voltages according to Example 4;
FIGS. 11A to 11D illustrate results of Example 5, illustrating the time courses and the Hct linearity provided when direct current voltages 0.5 V and 1.0 V were applied as the second signal to specimens in which the glucose value was 50 mg/dl and the Hct values were 0%, 10%, 42%, and 65% respectively;
FIGS. 12A to 12F illustrate the time courses and the Hct linearity provided when direct current voltages 1.5 V, 2.0 V, and 2.5 V were applied as the second signal to specimens in which the glucose value was 50 mg/dl and the Hct values were 0%, 10%, 42%, and 65% respectively;
FIGS. 13A to 13D illustrate the time courses and the Hct linearity provided when direct current voltages 0.5 V and 1.0 V were applied as the second signal to specimens in which the glucose value was 800 mg/dl and the Hct values were 0%, 10%, 42%, and 65% respectively; and
FIGS. 14A to 14F illustrate the time courses and the Hct linearity provided when direct current voltages 1.5 V, 2.0 V, and 2.5 V were applied as the second signal to specimens in which the glucose value was 800 mg/dl and the Hct values were 0%, 10%, 42%, and 65% respectively.

### DESCRIPTION OF EMBODIMENTS

Details of embodiments of the present disclosure are explained below. The measuring method according to an embodiment resides in a measuring method for measuring a target substance contained in a sample by using an analysis tool in which at least one working electrode provided with a reagent including an enzyme and at least one counter electrode are arranged in a flow passage. This measuring method comprises a step of applying a first signal, which is a direct current signal, having a first value to the sample disposed in the flow passage by using the working electrode and the counter electrode, a step of measuring a first electric response value of the sample depending on the first signal, a step of applying a second signal, which is a direct current signal, having a second value different from the first value to the sample disposed in the flow passage by using the working electrode and the counter electrode, a step of measuring a second electric response value of the sample at a point in time at which the application of the second signal continues for a given time after start thereof, and a step of correcting a value obtained from the first electric response value based on the second electric response value. The corrected value is dealt with as the measured value of the target substance contained in the sample.

The analysis tool is, for example, a biosensor. The sample is a liquid sample which is, for example, blood, interstitial fluid, or urine. The target substance contained in the sample is, for example, a glucose value (blood sugar value) or a lactate value (lactic acid value). The reagent may contain an enzyme and a mediator.

The analysis tool includes at least one working electrode. As for the number of the working electrode or working electrodes, one working electrode or a plurality of working electrodes may be used. If the analysis tool includes a plurality of working electrodes, the reagent is provided on the working electrode which is included in the plurality of working electrodes and which is used to measure the first electric response value and the second electric response value. For example, a solid reagent is fixed or immobilized on the working electrode.

In the measuring method according to the embodiment, it is preferable that the counter electrode, which is included in the at least one counter electrode and which is not provided with any salt component (a salt is included, for example, in an enzyme, a substrate, a mediator and a reagent), is used to apply the second signal. Further, in the present invention, it is preferable that the counter electrode, which is included in the at least one counter electrode and which is not provided with any mediator, is used to apply the second signal.

In the measuring method according to the embodiment, the working electrode, which is used to apply the first signal, is used as the working electrode which is used to apply the second signal. That is, the common working electrode is used for the measurement based on the use of the first signal and the measurement based on the use of the second signal. Accordingly, the first signal and the second signal can be measured in an identical measurement environment. Therefore, it is possible to improve the measurement accuracy. Further, when the working electrode is used in common, then it is possible to decrease the number of parts of the analysis tool, and it is possible to miniaturize the analysis tool. Further, it is possible to suppress the cost of the analysis tool.

In the measuring method according to the embodiment, it is preferable that when the analysis tool includes, as the at least one counter electrode, a first counter electrode which is provided with the reagent including the enzyme and a mediator and a second counter electrode which is not provided with the reagent including the enzyme and the mediator, then the first counter electrode is used to apply the first signal, and the second counter electrode is used to apply the second signal. That is, it is also allowable that the different counter electrodes are used for the measurement based on the use of the first signal and the measurement based on the use of the second signal. Accordingly, the first signal and the second signal can be applied in an identical measurement environment. Therefore, it is possible to improve the measurement accuracy based on the use of the first signal.

In the measuring method according to the embodiment, it is also allowable that the counter electrode, which is used to apply the first signal, is used to apply the second signal. That is, the common counter electrode is used for the measurement based on the use of the first signal and the measurement based on the use of the second signal. Accordingly, the first signal and the second signal can be measured in an identical measurement environment. Therefore, it is possible to improve the measurement accuracy. Further, when the common counter electrode is used, then it is possible to decrease the number of parts of the analysis tool, and it is possible to miniaturize the analysis tool. Further, it is possible to suppress the cost of the analysis tool.

In the embodiment, it is preferable that the counter electrode, which is included in the at least one counter electrode and which is not provided with the reagent including the enzyme and a mediator, is used to apply the first signal and apply the second signal, for the following reason. That is, if the reagent is provided, there is a possibility that the surrounding environment of the counter electrode may change on account of the reaction between the reagent and the sample, and the measurement result may be affected thereby.

In the embodiment, it is preferable that the sample is blood. Further, it is preferable that the target substance is glucose. It is preferable that the second electric response value of the sample is a value which indicates hematocrit. Further, it is preferable that the first electric response value of the sample is a value which indicates glucose value before being corrected by the value which indicates hematocrit.

In the embodiment, the measurement of the first electric response value and the measurement of the second electric response value are performed at different timings. As for the order of the measurement, any one may be performed earlier. In the present invention, the first signal has the first value and the second signal has the second value different from the first value. Each of the first signal and the second signal is, for example, a voltage whose direction does not change with time, namely a direct current voltage. The first signal may be called the first voltage and the second signal may be called the second voltage. The first value and the second value differ depending on the type of the mediator, the enzyme, and the electrode material. However, as the absolute condition, "first value < second value" is fulfilled (the second value is larger than the first value) .

A lower limit value of direct current voltage applied to electrodes, in order to cause electrolysis of water, is about 1.0 V in view of the voltage at which electrolysis of water occurs. When the direct current voltage is 1.0 V or more, an electric response value is not affected by the target substance in the sample. On the other hand, bubbles are generated in the sample as the applied voltage increases, and the electrical response value becomes a value influenced by bubbles. Therefore, an upper limit of the applied voltage is about 7.0V at which bubbles are not generated. For example, in a case of using ruthenium as a material of electrodes (using a ruthenium electrode), it is preferable that the lower limit of the applied voltage is 1.5 V and the upper limit of the applied voltage is 5.0 V. For example, when ruthenium is used for the electrode material and the counter electrode is not provided with the reagent, then the direct current voltage value as the first signal (example of the first value) is about 0.5 V (which is, for example, below a value of direct current voltage at which electrolysis of water occurs), and the direct current voltage value as the second signal (example of the second value) is about 2.5 V (which is, for example, above a value of direct current voltage at which electrolysis of water occurs). However, as mentioned above, the upper limit of the second value is about 7V at which bubbles are not produced. In the present invention, the second value of the direct current voltage, which is applied as the second signal, is, for example, 2.5 V, preferably from 1 V to 7 V, and more preferably from 1.8 V or 2 V to 5 V. The given time is, for example, 3 seconds, preferably from 2 to 5 seconds, and more preferably from 2.5 to 4 seconds. The first value of the direct current voltage as the first signal is the value which is smaller than the second value. The first value is, for example, 200 mV, preferably 50 mV to 1 V, and more preferably 100 mV to 500 mV. The above mentioned ranges of the first and second values are an example in a case where the material of electrodes is ruthenium. A range of an appropriate applied voltage varies depending on a type of enzyme and substrate, namely salt.

In the embodiment, the second signal, which is not less than the given value, is applied to the sample for not less than the given time. The second electric response value is measured at the point in time at which the application continues for the given time after the start thereof (i.e. at the point in time following the application of the second signal for the given time). This is based on the following measurement principle. The second signal having the second value (for example, direct current voltage) is applied to the sample disposed in the flow passage of the analysis tool (electrode for measuring the second electric response value). In this procedure, the second electric response value, which is provided in a period of about 1 second after the start of the application, exhibits an attenuation curve depending on the concentration of the target substance (for example, glucose value) contained in the sample. On the contrary, when about 2 seconds elapse after the start of the application, the second electric response value exhibits a constant value depending on the Hct value of the sample irrelevant to (independent of) the concentration of the target substance. Such a phenomenon occurs as follows.

That is, the charge transfer advances in an order of "target substance -> reagent -> electrode" in accordance with the application of the second signal. Further, the charge transfer occurs in an order of "H₂O -> electrode" in accordance with the electrolysis of water (H₂O) contained in the sample. However, the force, with which the electric charge transfers from H₂O to the electrode, is stronger than the force with which the electric charge transfers from the target substance via the reagent to the electrode. Note that when the reagent contains the enzyme and the mediator, the charge transfer advances in an order of "target substance -> reagent (enzyme -> mediator) -> electrode".

On this account, the second electric response value, which is caused by the charge transfer from the target substance, is observed in a certain degree of period (about 1 second) after the start of the application of the second signal. However, the charge transfer from H₂O to the electrode is greater than the charge transfer from the reagent to the electrode after the elapse of the given time (about 2 seconds). The reagent is saturated while holding the electric charge, and the charge transfer does not occur from the reagent to the electrode. That is, the second electric response value, which is provided in response to the application of the second signal, is substantially the value which indicates the electric charge amount generated by the electrolysis of H₂O, after the elapse of about 2 seconds (given time) after the start of the application of the second signal. In other words, the second electric response value is a value indicating amount of electric charge generated by electrolysis of water in the sample, and the second electric response value is obtainable by continuously applying the second signal for a given time. Note that when the reagent contains the enzyme and the mediator, the disappearance of the charge transfer from the reagent to the electrode means any one of the disappearance of the charge transfer from the enzyme to the mediator and the disappearance of the charge transfer from the mediator to the electrode and the both.

In this context, the hematocrit is the volume of the tangible component (blood cell or hemocyte) contained in blood. Therefore, the high Hct value means the small amount of H₂O (blood plasma) subjected to the electrolysis. Therefore, the higher the Hct value is, the lower the second electric response value is, the second electric response value being provided at the point in time at which the application continues for the given time. In other words, the second electric response value, which is provided (measured) at the point in time at which the application continues for the given time (i.e. at the point in time following the application of the second signal for the given time), is proportional to the Hct value in accordance with a negative function. Accordingly, if the calibration curve (for example, a table to indicate the correlation) is prepared beforehand between the second electric response value and the Hct value, it is possible to calculate the Hct value from the second electric response value provided (measured) at the point in time at which the application continues for the given time (i.e. after the application of the second signal for the given time). The second electric response value and the Hct value as described above can be used to correct the target substance.

According to the foregoing description, it is possible to understand the following fact. That is, the second electric response value, which exhibits the correct Hct value as compared with the second electric response value provided before the elapse of the given time, can be obtained by measuring the second electric response value provided at the point in time at which the application of the second signal continues for the given time after the start thereof (i.e. after the application of the second signal for the given time) . That is, it is possible to perform the highly accurate Hct value measurement. As a result, it is possible to raise the accuracy of the correction of the target substance. It is possible to perform the measurement of the target substance in which the accuracy is improved. Further, the electrochemical reaction, which is caused by the application of the second signal, is stabilized at the point in time at which the application continues for the given time as compared with the point in time which is provided before the elapse of the given time. Therefore, the Hct value having the high accuracy can be obtained from the second electric response current value, as compared with the Hct value which is determined from the second electric response current value provided before the elapse of the given time.

Here, a state that "reagent is provided on an electrode" includes a state that the regent is in contact with the electrode and is established or installed on the electrode, a state that the regent is fixed or immobilized on the electrode, a state that the regent is put, loaded, mounted, or disposed on the electrode, and the like. However, there is a case where although the reagent is not provided on the working electrode and the counter electrode, the reagent is provided between the working electrode and the counter electrode. The working electrode and the counter electrode in this case are included in "two electrodes used to measure a first electric response value". A state that "the reagent is provided between the working electrode and the counter electrode" means, for example, a state that the reagent is in contact with a substrate between the working electrode and the counter electrode and the reagent is established or installed on the substrate between the working electrode and the counter electrode. The state that "the reagent is provided between a working electrode and a counter electrode" includes, for example, a state that the reagent is in contact with a spacer and/or a cover and the reagent is established or installed on the spacer and/or the cover. Each of the spacer and the cover forms a flow passage between the working electrode and the counter electrode. This state includes, for example, a state that although there is no established or installed position of the reagent on a substrate, there is the reagent between the working electrode and the counter electrode in a planar view of the flow passage. In other words, a state that "the reagent is provided between a working electrode and a counter electrode" means that the established or installed reagent is provided in a state that the reagent diffuses (moves) to measurement environment in a vicinity of the working electrode due to the influence of the sample introduced into the flow passage.

### [Embodiment]

An explanation will be made below with reference to the drawings about the measuring method and the measuring apparatus according to the embodiments. The structure or arrangement of the embodiments explained below is referred to by way of example. The present disclosure is not limited to the structure or arrangement of the embodiments.

In the following embodiments, an explanation will be made about a measuring method and a measuring apparatus, as examples of the measuring method and the measuring apparatus, in which a biosensor as an analysis tool is used, the glucose value in blood is measured as a target substance contained in a sample, and the glucose value is corrected by using the Hct value of blood (second electric response value) . The present disclosure is not limited to the use of a biosensor as an analysis tool, nor to blood as a sample, nor to glucose as the target substance, nor to correcting on the basis of a Hct value.

### <Structure of biosensor>

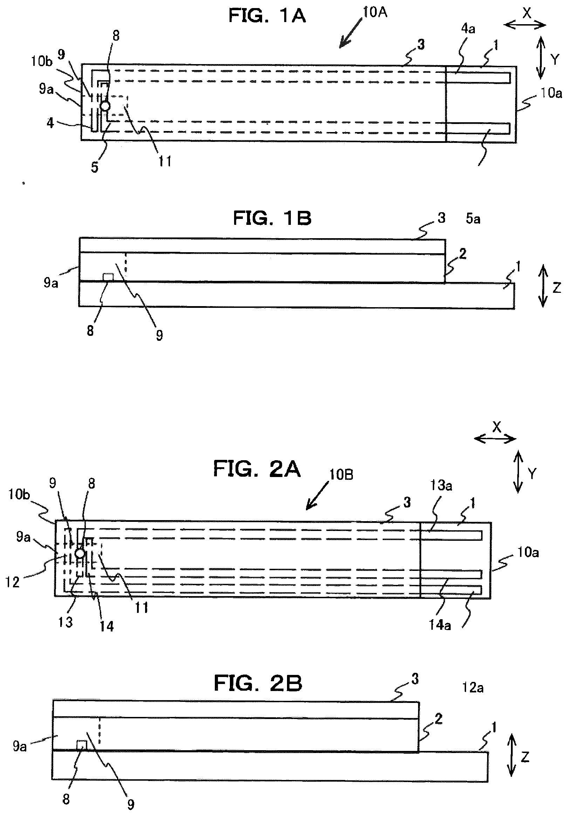

FIGS. 1A and 1B and FIGS. 2A and 2B illustrate exemplary structures of biosensors according to embodiments. FIG. 1A illustrates a top view illustrating an analysis tool (biosensor having two electrodes) according to the embodiment, and FIG. 1B illustrates a side view illustrating the biosensor illustrated in FIG. 1A. FIG. 2A illustrates a top view illustrating an analysis tool (biosensor having three electrodes) according to the embodiment, and FIG. 2B illustrates a side view illustrating the biosensor illustrated in FIG. 2A.

In FIG. 1A and FIG. 1B, the biosensor 10A (hereinafter referred to as "sensor 10A") has the longitudinal direction (X direction) having one end 10a and the other end 10b and the widthwise direction (Y direction). The sensor 10A is formed by stacking and adhering an insulating substrate 1 (hereinafter referred to as "substrate 1"), a spacer 2, and a cover 3 in the height direction (Z direction).

For example, a synthetic resin (plastic) is used for the substrate 1. As the synthetic resin, for example, it is possible to use various resins such as polyetherimide (PEI), polyethylene terephthalate (PET), polybutylene terephthalate (PBT), polyethylene
(PE), polystyrene (PS), polymethacrylate (PMMA), polypropylene (PP), polyimide resin, acrylic resin, epoxy resin, and glass epoxy. Note that any insulating material other than the synthetic resin can be used for the substrate 1. The insulating material includes, for example, paper, glass, ceramic, and biodegradable material, in addition to the synthetic resin. The material, which is the same as that of the substrate 1, can be used for the spacer 2 and the cover 3.

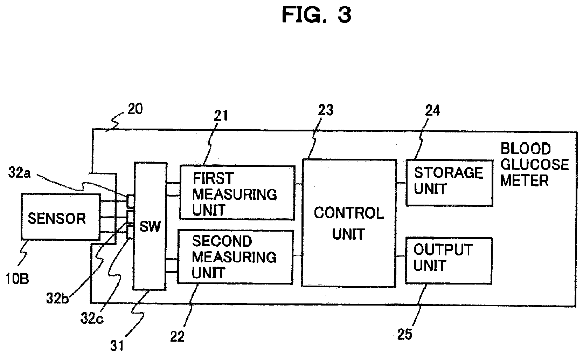

A first electrode 4 and a second electrode 5 are provided on the upper surface of the substrate 1 as one example of a plurality of electrodes. Each of the first electrode 4 and the second electrode 5 has a key-shape (for example, an L-shape) including a portion which extends in the widthwise direction of the sensor 10A and a portion which extends in the longitudinal direction of the sensor 10A. The portion, which extends in the longitudinal direction, is a lead portion 4a or a lead portion 5a. The lead portion 4a and the lead portion 5a, which are disposed on the one end 10a side, are not covered with the spacer 2 and the cover 3. The lead portion 4a and the lead portion 5a are used for the electric connection depending on a connector of a blood glucose meter 20 (FIG. 3).

Each of the first electrode 4 and the second electrode 5 is formed by using, for example, a metal material or an alloy thereof such as gold (Au), platinum (Pt), silver (Ag), palladium, ruthenium, and nickel or a carbon material such as carbon. For example, each of the first electrode 4 and the second electrode 5 can be formed as a metal layer having a desired thickness by forming a film of a metal material by means of the physical vapor deposition (PVD, for example, sputtering) or the chemical vapor deposition (CVD). Alternatively, each of the first electrode 4 and the second electrode 5 can be also formed by printing an ink containing a carbon material on the substrate 1 by means of the screen printing.

The spacer 2 has a cutout portion having a rectangular-shape (recess recessed from the other end 10b toward the one end 10a side) . The cutout portion opens toward the other end 10b side. By laminating or stacking the substrate 1, the spacer 2, and the cover 3, a space, which has an opening 9a formed by the cutout portion of the spacer 2, is formed on the other end 10b side of the sensor 10A. The space is formed by a thickness surface of the cutout portion, an upper surface of the substrate 1 on which the electrodes are provided, and an undersurface (lower surface) of the cover 3. The upper surface of the substrate 1 and the undersurface (lower surface) of the cover face each other into the space (not covered by bonding with the spacer 2). The space is used as a passage 9 for the sample. An air hole (air vent) 11 is formed through the cover 3. The flow passage 9 is formed so that the sample, which is spotted to the opening 9a, is attracted or drawn (introduced) into the flow passage in accordance with the capillary phenomenon and the sample is moved toward the air hole 11 (biological sample flows through the flow passage 9). Parts of the first electrode 4 and the second electrode 5 are exposed in the flow passage 9. A reagent 8 is provided (immobilized) on the second electrode 5. On the other hand, the reagent 8 is not provided on the first electrode 4. The reagent 8 may be either provided or not provided on the first electrode 4. Preferably, the reagent 8 is not provided on the first electrode 4.

The reagent 8 contains an enzyme and/or a substrate. In other words, the reagent 8 includes a salt. The reagent 8 may further contain a mediator. The enzyme is appropriately selected depending on the type of the sample and the target substance. When the target substance is glucose contained in blood or interstitial fluid, glucose oxidase (GOD) or glucose dehydrogenase (GDH) is applied. The mediator is, for example, ferricyanide, ruthenium complex, p-benzoquinone, p-benzoquinone derivative, phenazine methosulfate, methylene blue, ferrocene, ferrocene derivative, and ruthenium complex. Among them, it is preferable to apply ferricyanide or ruthenium complex, and it is more preferable to apply potassium ferricyanide or ruthenium compounds [Ru(NH₃)₆]cl₃.

The first electrode 4 and the second electrode 5 are used as the electrode pair to be used for the measurement of the glucose value and the measurement of the Hct value. The first electrode 4 is used as the counter electrode in both of the measurement of the glucose value and the measurement of the Hct value. The second electrode 5, on which the reagent 8 is provided, is used as the working electrode in both of the measurement of the glucose value and the measurement of the Hct value. In this way, in the case of the sensor 10A, the counter electrode and the working electrode are commonly used between the measurement of the glucose value and the measurement of the Hct value.

The biosensor 10B illustrated in FIG. 2A and FIG. 2B has a three-electrode structure including a first electrode 12, a second electrode 13, and a third electrode 14 in place of the first electrode 4 and the second electrode 5. The reagent 8 is provided on the second electrode 13. On the other hand, the reagent 8 is not provided on the first electrode 12 and the third electrode 14. That is, the reagent 8 may be either provided or not provided on the first electrode 12 and the third electrode 14. Preferably, any one of the salt component, the mediator, and the reagent 8 is not provided on the first electrode 12.

The first electrode 12 is used as the counter electrode in the measurement of the Hct value. The second electrode 13, on which the reagent 8 is provided, is used as the working electrode in both of the measurement of glucose and the measurement of the Hct value. In this way, the working electrode is commonly used between the measurement of the glucose value and the measurement of the Hct value. Further, an unillustrated reagent (having the same components as the components explained for the reagent 8) is provided on the third electrode 14. The third electrode 14 is used as the counter electrode for measuring the glucose value. Except for the above, the sensor 10B is constructed in the same manner as the sensor 10A.

The third electrode 14 of the sensor 10B is used as the electrode for measuring the glucose value, for which it is possible to adopt such a structure that the reagent is not provided. Further, the biosensor (for example, the sensor 10B) may have a four-electrode structure having an electrode (not illustrated) other than the first electrode 12, the second electrode 13, and the third electrode 14.

In the case of the four-electrode structure, two electrodes and/or three electrodes of the four electrodes can be used to perform the measurement of the glucose value and the measurement of the Hct value in accordance with the same method of use as those of the sensor 10A and the sensor 10B described above. Further, in the case of the four-electrode structure, it is also allowable to use different electrode pairs between the measurement of the glucose value and the measurement of the Hct value. Even in this case, a reagent, which is equivalent to the reagent 8, is immobilized respectively to the electrode which is used as the working electrode in the measurement of the glucose value and the electrode which is used as the working electrode in the measurement of the Hct value. On the other hand, the reagent may be either provided or not provided respectively on the electrode which is used as the counter electrode in the measurement of the glucose value and the electrode which is used as the counter electrode in the measurement of the Hct value. Preferably, the reagent is provided on the electrode which is used as the counter electrode in the measurement of the glucose value, and the reagent is not provided on the electrode which is used as the counter electrode in the measurement of the Hct value.

The biosensor may further comprise an electrode which is used as a reference electrode and an electrode which is used to measure any measurement item other than the measurement of the glucose value and the measurement of the Hct value, in addition to the counter electrode and the working electrode concerning the measurement of the glucose value and the measurement of the Hct value described above.

### <Arrangement of measuring apparatus (blood glucose meter)>

FIG. 3 illustrates a block diagram illustrating an exemplary arrangement of the blood glucose meter 20 as an example of the measuring apparatus. With reference to FIG. 3, for example, the sensor 10B (FIGS. 2A and 2B) is connectable to the blood glucose meter 20. The blood glucose meter 20 measures the glucose value by using the connected sensor 10B, and the blood glucose meter 20 corrects the glucose value by using the Hct value.

The blood glucose meter 20 is provided with a first measuring unit 21, a second measuring unit 22, a switch (SW) 31, a control unit 23, a storage unit 24, and an output unit 25. The switch 31 is electrically connected to a connector 32a, a connector 32b, and a connector 32c. The connector 32a is connected to the lead portion 12a (first electrode 12) of the sensor 10B, the connector 32b is connected to the lead portion 13a (second electrode 13) of the sensor 10B, and the connector 32c is connected to the lead portion 14a (third electrode 14) of the sensor 10B.

The switch 31 is the switch which changes the states of electric connection and disconnection thereof with respect to the connector 32a, the connector 32b, and the connector 32c. The control unit 23 is a processor (for example, Central Processing Unit (CPU)) which executes the program stored in the storage unit 24. The storage unit 24 includes a memory which includes a main storage device such as RAM (Random Access Memory) or the like and an auxiliary storage device such as ROM (Read Only Memory), a hard disk or the like. The storage unit 24 stores, for example, the program which is to be executed by the control unit 23 and the data which is to be used when the program is executed. The output unit 25 includes output devices such as a printer, a display device and the like, and communication devices such as a signal connector, a communication interface and the like.

The control unit 23 controls the switch 31 during the measurement of the glucose value so that the working electrode (second electrode 13) and the counter electrode (third electrode 14) for measuring the glucose value are in the states of being electrically connected to the blood glucose meter 20 and the first electrode 12 is in the disconnected state. The first measuring unit 21 is configured by circuit (s) and/or a processor and a memory and performs operations below. That is, the first measuring unit 21, according to an instruction of the control unit 23, starts application of the direct current voltage as the first signal between the second electrode 13 and the third electrode 14. After the direct current voltage is continuously applied for a given time, the first measuring unit 21 measures a response current value corresponding to a glucose value (the first electric response value corresponding to the first signal) as a response current value corresponding to the direct current voltage. For example, the first measuring unit 21 measures, as a first electric response value, a response current value at an end point of the given time. However, a time point or timing to obtain the first electric response value may be a given time point other than the end point of the application of the first signal (a given time point in applying of the first signal).

The control unit 23 controls the switch 31 during the measurement of the Hct value so that the working electrode (second electrode 13) and the counter electrode (first electrode 12) for measuring the Hct value are in the states of being electrically connected to the blood glucose meter 20 and the third electrode 14 is in the disconnected state. The second measuring unit 22 is configured by circuit (s) and/or a processor and a memory and performs operations below. That is, the second measuring unit 22, according to an instruction of the control unit 23, applies the direct current voltage corresponding to the second signal between the second electrode 13 and the first electrode 12. After the direct current voltage is continuously applied for a given time, the second measuring unit 22 measures, as the second electric response value, a response current value indicating amount of electric charge generated by electrolysis of water in the sample. However, a time point or timing to obtain the second electric response value may be a given time point other than the end point of the application of the second signal (a given time point in applying of the second signal).

Note that when the blood glucose meter 20 has an arrangement corresponding to the sensor 10A (FIG. 1), then the connector 32c is omitted, the connector 32a is connected to the lead portion 4a (first electrode 4), and the connector 32b is connected to the lead portion 5a (second electrode 5). The switch 31 is in the state in which the first measuring unit 21 is electrically connected to the first electrode 4 and the second electrode 5, during the measurement of the glucose value, for example, by means of the control effected from the control unit 23.

On the contrary, the switch 31 is in the state in which the second measuring unit 22 is electrically connected to the first electrode 4 and the second electrode 5, during the measurement of the Hct value, for example, by means of the control effected from the control unit 23. In this way, the connection state is switched for the first electrode 4 and the second electrode 5 between the first measuring unit 21 and the second measuring unit 22. The first measuring unit 21 applies the direct current voltage as the first signal between the first electrode 4 and the second electrode 5 to measure the first electric response value. The second measuring unit 22 applies the direct current voltage as the second signal between the first electrode 4 and the second electrode 5 to perform the measurement of the second electric response value after the elapse of the given time after the start of the application.

The storage unit 24 stores the calibration curve data (calibration curve table) for determining the glucose value from the first electric response value measured by the first measuring unit 21. The control unit 23 converts the first electric response value into the glucose value by using the calibration curve table, and thus the control unit 23 calculates the glucose value. The glucose value is stored in the storage unit 24 in some cases, and/or the glucose value is outputted (for example, displayed) from the output unit 25 in other cases. A calibration curve table may be used to obtain the glucose value instead of the calibration curve data.

Further, the storage unit 24 stores the calibration curve data for determining the Hct value corresponding to the value of the second electric response value measured by the second measuring unit 22. The control unit 23 converts the second electric response value into the Hct value by using the calibration curve table, and thus the control unit 23 calculates the Hct value. The Hct value is stored in the storage unit 24 in some cases, and/or the Hct value is outputted (for example, displayed) from the output unit 25 in other cases. A calibration curve table may be used to obtain the glucose value instead of the calibration curve data.

Further, the control unit 23 performs a process (Hct correction of the glucose value) in which the glucose value is corrected by using the second electric response value obtained from the second measuring unit 22 or the Hct value obtained from the second electric response value. For example, the storage unit 24 stores the calibration curve data or the calibration curve table) which represents the correlation or corresponding relationship between the second electric response value and the amount of correction. The control unit 23 determines the amount of correction corresponding to the second electric response value by using the calibration curve data or the calibration curve table. The control unit 23 performs the process for correcting the glucose value, and the control unit 23 calculates the corrected glucose value. The corrected glucose value is stored in the storage unit 24 in some cases, and/or the corrected glucose value is outputted (for example, displayed) from the output unit 25 in other cases. Note that when the second electric response value is used to correct the glucose value, it is not necessarily essential to perform the conversion from the second electric response value to the Hct value. The control unit 23 may perform correction of the second electric response value based on a temperature of measurement environment of the second electric response value using the calibration curve data or a calibration curve table for the correction.

### <Exemplary operation>

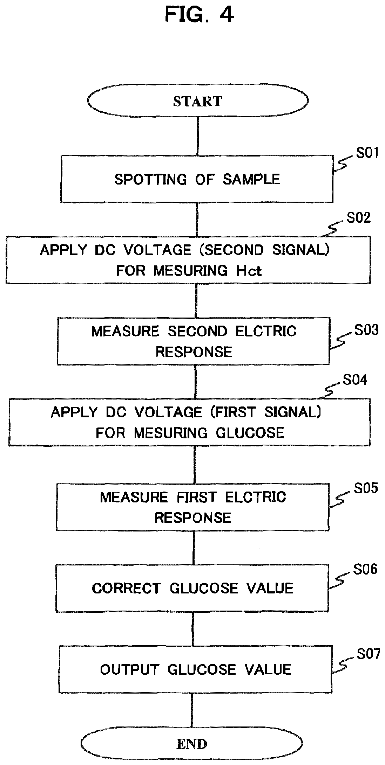

FIG. 4 illustrates a flow chart illustrating an exemplary operation of the blood glucose meter 20. In S01 illustrated in FIG. 4, the spotting of the sample is performed. That is, when the biosensor (for example, sensor 10B) is connected to the blood glucose meter 20, and the opening 9a of the other end 10b of the sensor 10B is brought in contact (subjected to the spotting) with the blood (biological sample) collected from an examinee, then the blood is drawn (attracted) into the flow passage 9 in accordance with the capillary force, and the interior of the flow passage 9 is filled therewith.

The control unit 23 applies the voltage to the electrode before the spotting to observe the applied voltage. The control unit 23 detects the change of the voltage caused by the contact between the blood and the electrode as a result of the spotting, and the control unit 23 detects that the blood is introduced into the flow passage 9. Then, the control unit 23 starts the measurement of the Hct value (S02) .

In S02, the control unit 23 controls the second measuring unit 22 to apply the second signal to the blood. That is, the control unit 23 gives the instruction to the second measuring unit 22 so that the direct current voltage (for example, 2.5 V) corresponding to the second signal is applied to the electrode pair (first electrode 12, second electrode 13) for measuring the Hct value.

The second measuring unit 22 waits until the application of the direct current voltage corresponding to the second signal continues for the given time (about 2 seconds) after the start thereof, and the second measuring unit 22 measures the second electric response of the blood at the point in time at which the application continues for the given time, namely a response value does not include amount of electric charge based on the reagent and indicates amount of electric charge generated by electrolysis of water in the sample (S03) . For example, the second measuring unit 22 measures the response signal depending on the direct current voltage, and the response signal is subjected to the A/D conversion and sent to the control unit 23.

When the control unit 23 acquires the second electric response of the blood depending on the second signal, the control unit 23 starts the measurement of the glucose value. That is, the control unit 23 applies the first signal to the blood (S04). That is, the control unit 23 instructs the first measuring unit 21 that the direct current voltage as the first signal, which is lower than the second signal, is applied to the electrode pair (third electrode 14, second electrode 13) for measuring the glucose value. The first measuring unit 21 applies the direct current voltage in accordance with the instruction.

The first measuring unit 21 measures the first electric response of the blood depending on the first signal (S05). For example, the first measuring unit 21 measures the response current of the first signal. The second measuring unit 22 performs the A/D conversion of the response current to be sent to the control unit 23.

The control unit 23 performs the correcting process for the glucose value (S06) . That is, the control unit 23 calculates the value of the correction object component contained in the blood (glucose value) by using the first electric response acquired in S05, the calibration curve data or the calibration curve table. Further, the control unit 23 calculates the corrected glucose value by using the second electric response acquired in S03 (or the Hct value corresponding to the second electric response).

The control unit 23 outputs the corrected glucose value (S07) . That is, the control unit 23 stores the glucose value corrected in S06 in the storage unit 24, and the glucose value is displayed on the output unit 25 (display) . The control unit 23 can also transmit the glucose value to any other device via any wired or wireless network by using the output unit 25.

### [Example 1]

In Example 1, the measurement of the Hct value and the measurement of the glucose value were performed for a specimen by using a working electrode provided with a reagent and a counter electrode not provided with any reagent. In Example 1, as for the biosensor, a biosensor having the structure of the sensor 10A was manufactured, in which a first electrode 4 and a second electrode 5 were made of ruthenium. The reagent 8 was provided on the second electrode 5. The formulation and the production method of the reagent 8 are as follows. An enzyme solution was prepared, which contained polyvinyl alcohol (PVA 146,000) 0.8% by weight, ruthenium compound [Ru(NH₃)₆]Cl₃ 1.6% by weight, FAD-GDH 2.7 U, 1-methoxy PMS 0.3% by weight, and ACES buffer (pH 6.5). 0.5 µL of the enzyme solution was dispensed, followed by being dried at 25°C to thereby obtain the reagent 8.

Whole bloods of two people were mixed and used to prepare specimens. A plurality of specimens, in which the Hct value was adjusted to 0%, 20%, 42%, and 65% and the glucose value was adjusted to 50 mg/dl, 400 mg/dl, and 800 mg/dl respectively, were prepared.

The Hct value and the glucose value were measured under the following conditions.

### (1) Measurement of second electric response value (Hct value)

The application of the direct current voltage (2.5 V: corresponding to the second signal having the second value) for measuring the Hct value was started 0.5 second (0.5 second open circuit) after connecting, to the measuring apparatus, the Hct value measuring electrodes (first electrode 4 and second electrode 5) of the biosensor in which the specimen was introduced into the flow passage. The application of the direct current voltage was continued for 2 seconds, and the response current (second electric response value) was measured 2 seconds after the start of the application (at the point in time at which 2.5 seconds elapsed after the start of the measurement).

### (2) Measurement of first electric response value (glucose value)

The application was stopped 2 seconds after the start of the application of direct current voltage 2.5 V (second signal), and a state of open circuit (no electric power-applied state) was given for 1 second. After that, the direct current voltage for measuring the glucose value (1000 mV: corresponding to the first signal having the first value) was applied. Then, the response current (first electric response value) was measured at the point in time of 7 seconds after the start of the application of the first signal. In this way, the measurement of the Hct value and the measurement of the glucose value were continuously performed for the same specimen (biological sample).

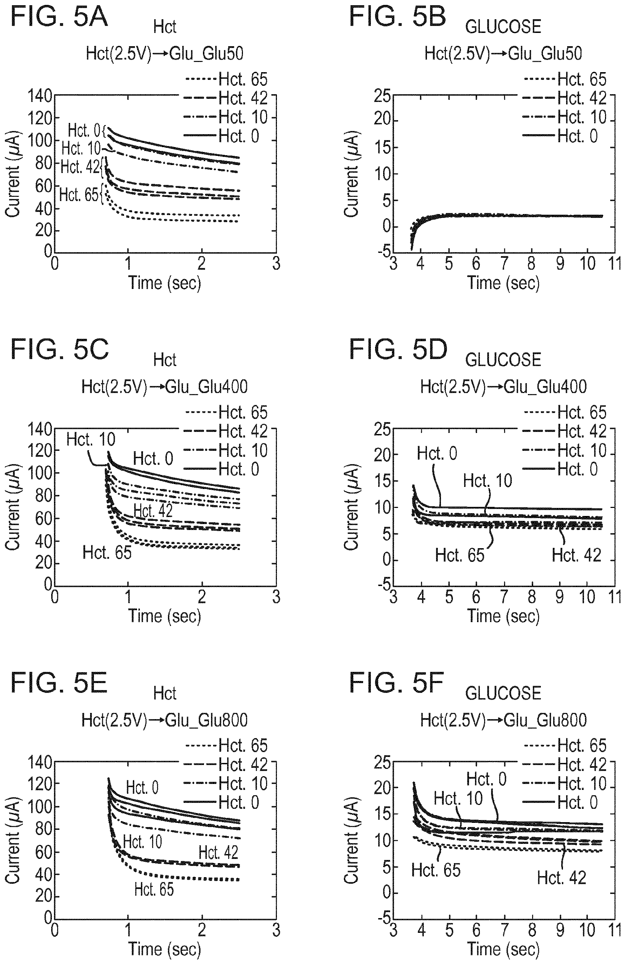

FIGS. 5A, 5B, 5C, 5D, 5E and 5F illustrate results of the measurement of the Hct value and the glucose value in Example 1. FIG. 5A illustrates the result of the measurement of the Hct value carried out under the measurement condition of (1) described above for the specimens in which the glucose value was 50 mg/dl and the Hct values were 0%, 20%, 42%, and 65% respectively. FIG. 5B illustrates the result of the measurement of the glucose value carried out under the measurement condition of (2) described above continuously to the measurement of the Hct value concerning FIG. 5A. FIG. 5C illustrates the result of the measurement of the Hct value carried out under the measurement condition of (1) described above for the specimens in which the glucose value was 400 mg/dl and the Hct values were 0%, 20%, 42%, and 65% respectively. FIG. 5D illustrates the result of the measurement of the glucose value carried out under the measurement condition of (2) described above continuously to the measurement of the Hct value concerning FIG. 5C. FIG. 5E illustrates the result of the measurement of the Hct value carried out under the measurement condition of (1) described above for the specimens in which the glucose value was 800 mg/dl and the Hct values were 0%, 20%, 42%, and 65% respectively. FIG. 5F illustrates the result of the measurement of the glucose value carried out under the measurement condition of (2) described above continuously to the measurement of the Hct value concerning FIG. 5E.

FIG. 5A, FIG. 5C, and FIG. 5E illustrate the time-dependent changes of the response current as the second electric response provided when the second signal (direct current voltage 2.5 V) was applied. FIG. 5B, FIG. 5D, and FIG. 5F illustrate the time-dependent changes of the response current as the first electric response provided when the first signal (direct current voltage 1.0 V) was applied.

With reference to FIG. 5A, FIG. 5C, and FIG. 5E, the current value suddenly decreases irrelevant to (irrespective of) the glucose value and the Hct value at about 1 second after the start of the measurement in the waveform (time course) of each of the time-dependent changes. This is considered to be caused by a primary factor of the charge transfer from glucose via the reagent (enzyme and mediator) to arrive at the electrode. The response current, which is provided at this point in time, depends on the glucose value. On the contrary, the inclination of the current is gentle or substantially constant after the elapse of about 2 seconds (at the point in time at which the application continues for about 2 seconds), probably for the following reason. That is, it is considered that the response current resides in (results from) the charge transfer from H₂O (blood plasma) contained in the sample to the electrode (response current depends on the Hct value) as described above.

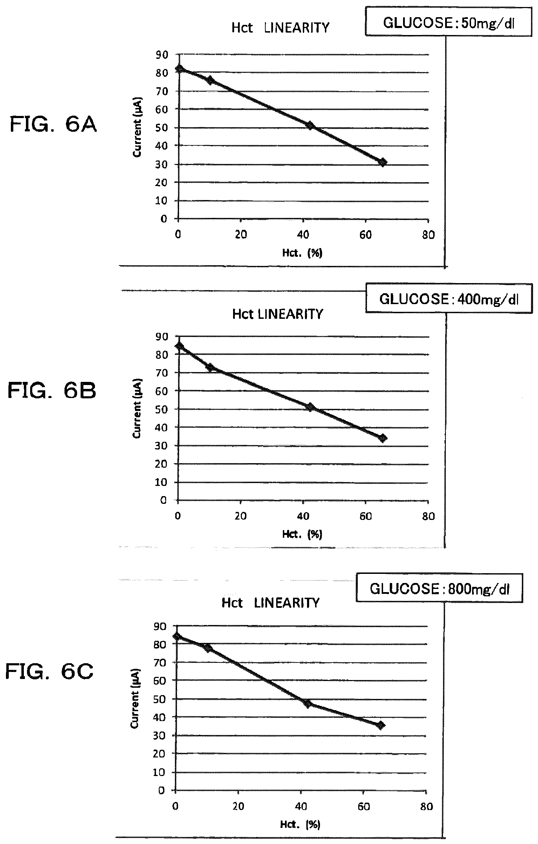

FIGS. 6A to 6C illustrate the Hct linearity according to Example 1. FIG. 6A illustrates the linearity of the Hct value (relationship between the response current and the Hct value) provided when the glucose value is 50 mg/dl. FIG. 6B illustrates the linearity of the Hct value (relationship between the response current and the Hct value) provided when the glucose value is 400 mg/dl. FIG. 6C illustrates the linearity of the Hct value (relationship between the response current and the Hct value) provided when the glucose value is 800 mg/dl.

In any one of FIG. 6A, FIG. 6B, and FIG. 6C, the value of the response current (second electric response), which was measured 2.5 seconds after the start of the measurement under the measurement condition of (1) described above (2 seconds after the start of the application), was used as the Hct value. According to the results illustrated in FIG. 6A, FIG. 6B, and FIG. 6C, it is possible to confirm that the response current value and the Hct value are correlated with each other irrelevant to the glucose value. According to Example 1, the following fact is comprehended. That is, the current, which depends on the Hct value, can be measured by measuring the response current 2 seconds after the start of the application of direct current voltage 2.5 V.

### [Example 2]

In Example 2, the Hct value and the glucose value were measured when the biosensor 20B (see FIG. 2A and FIG. 2B) having the three-electrode structure was used as the biosensor. In Example 2, the sensor 10B (three-electrode structure), in which ruthenium was used for the electrode material, was prepared, and the reagent 8 was provided on the second electrode 13. When the Hct value was measured, then the first electrode 12 was used as the counter electrode, and the second electrode 13 was used as the working electrode. On the contrary, when the glucose value was measured, then the second electrode 13 was used as the working electrode, and the third electrode 14 was used as the counter electrode. A reagent (not illustrated), which was the same as or equivalent to the reagent 8, was provided on the third electrode 14. The formulation and the production method of the reagent 8 are the same as those of Example 1.

As for the specimens relating to Example 2, which had the same combinations of the Hct values and the glucose values as those of Example 1, were prepared.

Also in Example 2, the measurement of the glucose value was performed for the same specimen following the measurement of the Hct value. The measurement conditions (measuring methods) for the Hct value and the glucose value were as follows.

### (1) Measurement of second electric response value (Hct value)

The condition was the same as that of Example 1.

### (2) Measurement of first electric response value (glucose value)

The application was stopped 2 seconds after the start of the application of direct current voltage 2.5 V (second signal), and a state of open circuit (no electric power-applied state) was given for 15 seconds. During this process, the electrode connection state was switched from the connection between the first electrode 12 and the second electrode 13 to the connection between the second electrode 13 and the third electrode 14. The application of the direct current voltage for measuring the glucose value (200 mV: corresponding to the first signal having the first value) was started 17.5 seconds after the start of the measurement. Then, the response current (first electric response value) was measured at the point in time of 7 seconds after the start of the application of the first signal.

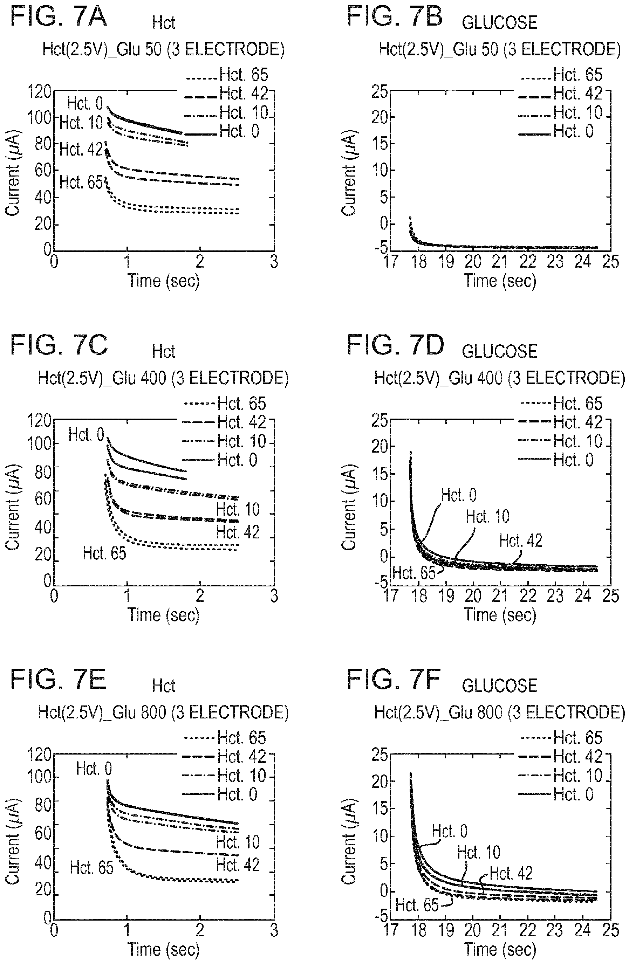

FIGS. 7A to 7F illustrate results of the measurement of the Hct value and the glucose value concerning Example 2. FIG. 7A illustrates the result of the measurement of the Hct value carried out for the specimens in which the glucose value was 50 mg/dl and the Hct values were 0%, 20%, 42%, and 65% respectively. FIG. 7B illustrates the result of the measurement of the glucose value carried out following the measurement of the Hct value concerning FIG. 7A. FIG. 7C illustrates the result of the measurement of the Hct value carried out for the specimens in which the glucose value was 400 mg/dl and the Hct values were 0%, 20%, 42%, and 65% respectively. FIG. 7D illustrates the result of the measurement of the glucose value carried out following the measurement of the Hct value concerning FIG. 7C. FIG. 7E illustrates the result of the measurement of the Hct value carried out for the specimens in which the glucose value was 800 mg/dl and the Hct values were 0%, 20%, 42%, and 65% respectively. FIG. 7F illustrates the result of the measurement of the glucose value carried out following the measurement of the Hct value concerning FIG. 7E.

With reference to the respective time courses of FIG. 7A, FIG. 7C, and FIG. 7E, the sudden decrease in the current value was observed irrelevant to (irrespective of) the glucose value and the Hct value at about 1 second after the start of the measurement, in the same manner as in Example 1. On the contrary, the inclination of the current was gentle or substantially constant after the elapse of about 2 seconds (at the point in time at which the application (of the second signal) continued for about 2 seconds).

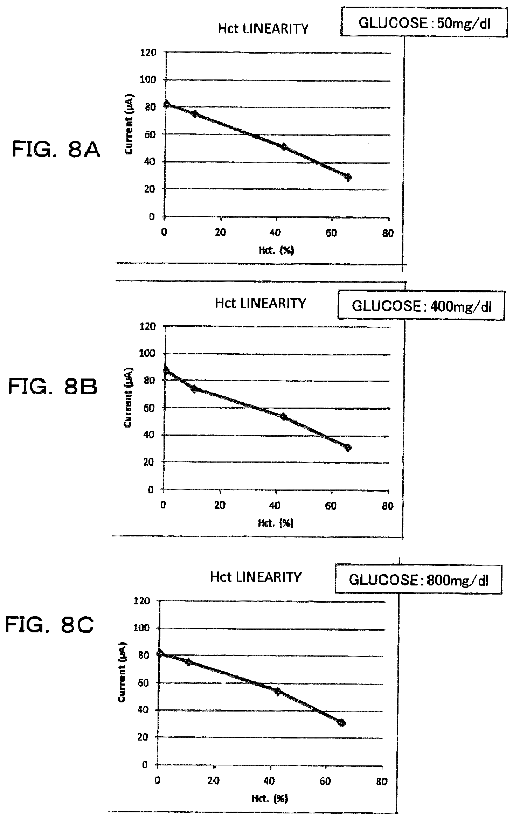

FIGS. 8A to 8C illustrate the Hct linearity according to Example 2. FIG. 8A illustrates the linearity of the Hct value provided when the glucose value is 50 mg/dl. FIG. 8B illustrates the linearity of the Hct value provided when the glucose value is 400 mg/dl. FIG. 8C illustrates the linearity of the Hct value provided when the glucose value is 800 mg/dl.

Also in Example 2, the value of the response current (second electric response), which was measured 2.5 seconds after the start of the measurement (2 seconds after the start of the application), was used as the Hct value. According to the results illustrated in FIG. 8A, FIG. 8B, and FIG. 8C, it is possible to confirm that the response current value and the Hct value are correlated with each other irrelevant to (irrespective of) the glucose value. According to Example 2, the following fact is comprehended. That is, it is possible to measure the current, which depends on the Hct value, by measuring the response current at the point in time at which the application of direct current voltage 2.5 V continues for 2 seconds after the start thereof.

### [Example 3]

In Example 3, a biosensor, in which palladium was used for the electrode material in place of ruthenium, was manufactured. The Hct value was measured under the same condition as that of Example 2. However, two types of the glucose values of the samples of 50 mg/dl and 800 mg/dl were used. Further, direct current voltages 2.5V, 3.5 V, 5.0 V, and 7.5 V were adopted as the applied voltage of the second signal.

Table 1 illustrated below illustrates the measurement results of the Hct value obtained when the respective applied voltages were applied for the respective specimens in which the glucose value was 50 mg/dl and the Hct values were 0%, 10%, 42%, and 65% and the specimen in which the glucose value was 800 mg/dl and the Hct value was 42%.

**[Table 1]**

| GLUCOSE (mg/dl) | 50 | | | | 800 |
|---|---|---|---|---|---|
| Hct (%) | 65 | 42 | 10 | 0 | 42 |
| 2.5V | 6.67268 | 8.56344 | 11.1269 | 12.4122 | 26.6908 |
| 3.5V | 28.5873 | 45.0345 | 65.2231 | 77.5712 | 45.72 |
| 5V | 84.7489 | 129.276 | 179.679 | 219.264 | 123.985 |
| 7.5V | 110.099 | 123.362 | 176.75 | 157.714 | 108.778 |

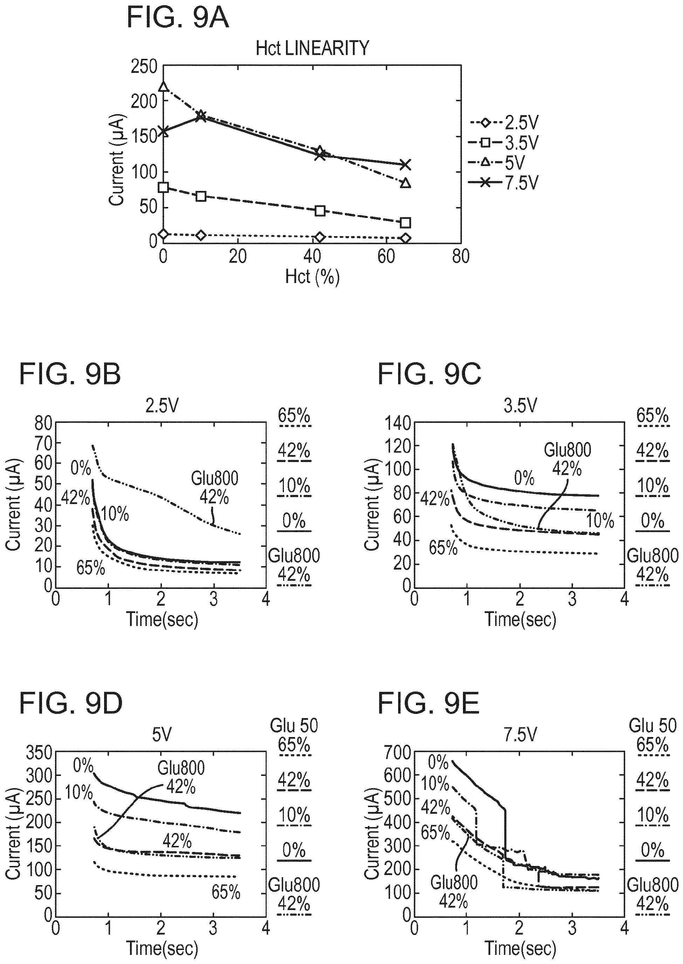

FIGS. 9A to 9E illustrate the Hct linearity and the time courses of the applied voltages according to Example 3. FIG. 9A illustrates a graph illustrating the Hct linearity corresponding to each of the applied voltages as the second signal. FIGS. 9B to 9E illustrate the relationships (time courses) between the response current (second electric response) and the time provided when the respective applied voltages were applied.

According to the graph of the Hct linearity illustrated in FIG. 9A, when the second signal (applied direct current voltage) was voltage 2.5 V, the second electric response depending on the glucose value was obtained. When the second signal was 3.5 V and when the second signal was 5.0V, then the second electric response depending on the Hct value was obtained. Note that when the applied direct current voltage was 7.5 V, any preferred Hct linearity was not obtained. This situation was caused by the breakage (exfoliation) of the counter electrode for measuring Hct and the disturbance of the time course.

### [Example 4]

In Example 4, a biosensor, in which carbon was used for the electrode material in place of ruthenium, was manufactured. The Hct value was measured under the same condition as that of Example 2. The glucose value of the sample and the applied voltage value of the second signal were the same as those used in Example 3.

Table 2 illustrated below illustrates the measurement results of the Hct value obtained when direct current voltages 2.5 V, 3.5 V, 5.0 V, and 7.5 V were applied as the applied voltage respectively for the respective specimens in which the glucose value was 50 mg/dl and the Hct values were 0%, 10%, 42%, and 65% and the specimen in which the glucose value was 800 mg/dl and the Hct value was 42%.

**[Table 2]**

| GLUCOSE (mg/dl) | 50 | | | | 800 |
|---|---|---|---|---|---|
| Hot (%) | 65 | 42 | 10 | 0 | 42 |
| 2.5V | 1.51 | 1.84 | 2.05 | 266 | 16.76 |
| 3.5V | 1099 | 13.71 | 19.89 | 17.78 | 20.63 |
| 5V | 6790 | 8874 | 117.44 | 123.38 | 87.95 |
| 7.5V | 141.87 | 220.95 | 286.90 | 289.60 | 217.73 |

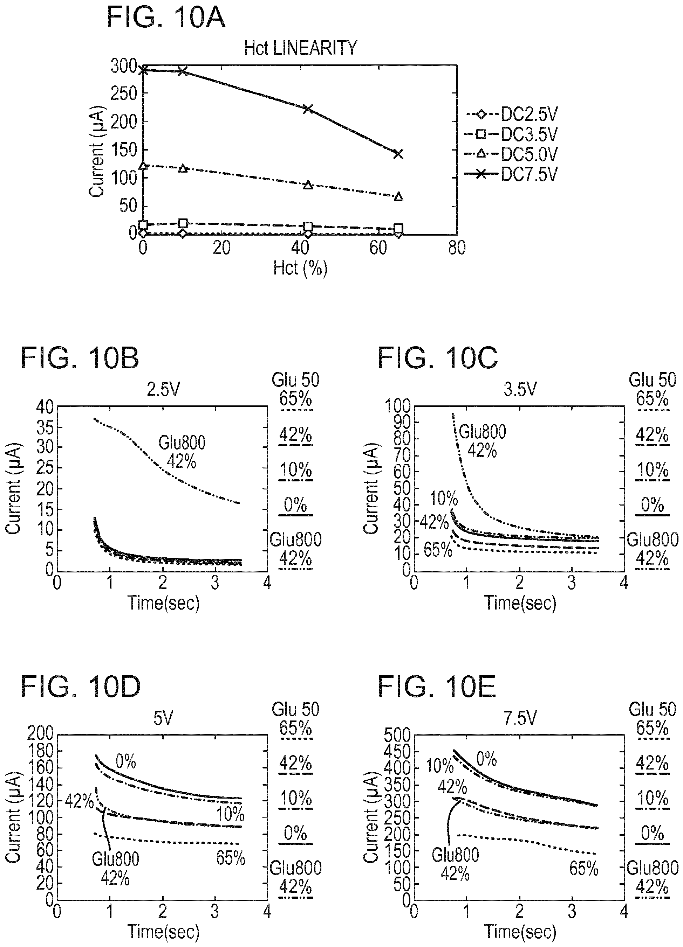

FIGS. 10A to 10E illustrate the Hct linearity and the time courses of the respective applied voltages according to Example 4. FIG. 10A illustrates a graph illustrating the Hct linearity corresponding to each of the applied voltages as the second signal. FIGS. 10B to 10E illustrate the relationships (time courses) between the response current (second electric response) and the time provided when the respective applied voltages are applied.

As illustrated in FIG. 10A, when the applied direct current voltage is 2.5 V and 3.5 V, the second electric response (response current) depends on the glucose value. On the contrary, when the applied direct current voltage was 5.0 V, the second electric response depending on the Hct value was observed. Even when the applied direct current voltage was 7.5 V, the second electric response depending on the Hct value was observed. Note that when the applied direct current voltage was 7.5 V, if the Hct value was 0% and 10%, then bubbles were observed, which were produced to such an extent that almost all flow passage was occupied thereby.

As described in Example 1 and Example 2, when ruthenium was used for the electric material, the response current (second electric response) depending on the Hct value was observed at the applied direct current voltage of 2.5 V. On the contrary, as described in Example 3 and Example 4, when the electrode material is palladium or carbon, the second electric response depending on the Hct value was not observed even when the applied direct current voltage of 2.5 V was applied.

The reason is considered as follows. It has been reported that the dissociation with respect to water molecule on the metal surface resides in "Au < Ag < Cu < Pd < Rh < Ru < Ni" (Surface Chemistry Vol. 30, No. 3, 130-1392009, "Relationship between water structure and electrode potential on electrode surface"). Accordingly, it is considered that the electrolysis of water occurs at a low electric potential in the case of the metal on which the dissociation of water molecule is caused with ease, and the reaction, which does not depend on the glucose value, is observed. Note that the reaction can be confirmed even when the mediator and NaCl (one example of the salt component) are provided on the working electrode, and it is considered that the measurement of the Hct value can be performed on condition that a certain amount of salt is supplied.

### [Example 5]

In Example 5, an experiment was performed in order to investigate the lower limit of the second signal (direct current voltage) to measure the Hct value. As for the biosensor in which ruthenium was used for the electrode material, a biosensor (corresponding to the sensor 10A) having a two-electrode structure was prepared, in which the reagent was provided on the working electrode and the reagent was not provided on the counter electrode.

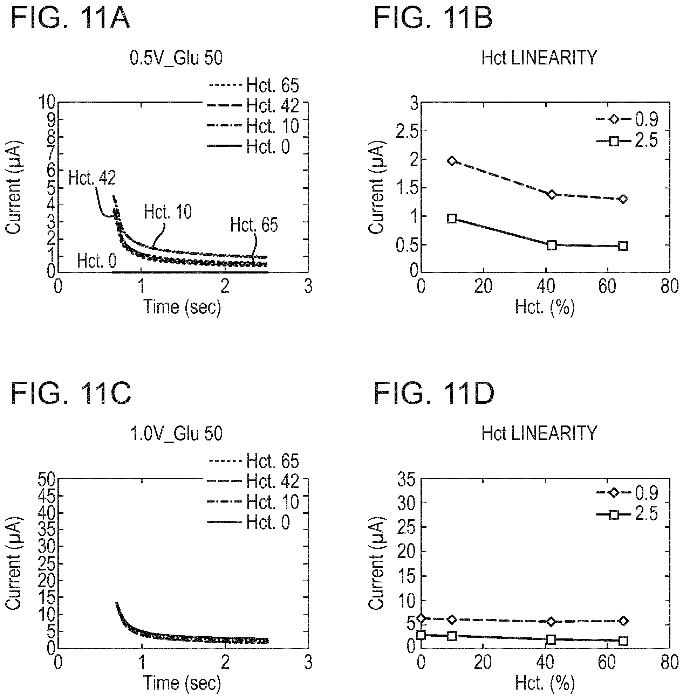

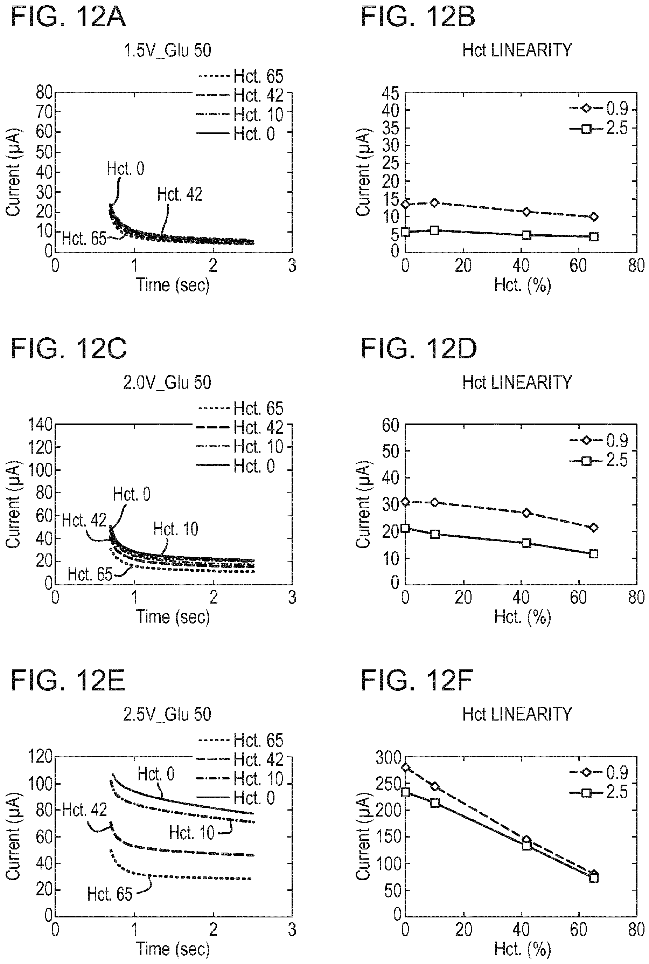

FIGS. 11A to 11D and FIGS. 12A to 12D illustrate the time courses and the Hct linearity provided when direct current voltages 0.5 V, 1.0 V, 1.5 V, 2.0 V, and 2.5 V were applied as the second signal to samples in which the glucose value was 50 mg/dl and the Hct values were 0%, 10%, 42%, and 65% respectively. Specifically, FIG. 11A and FIG. 11B illustrate the time course and the Hct linearity obtained when the direct current voltage 0.5 V was applied to the sample in which the glucose value was 50 mg/dl. FIG. 11C and FIG. 11D illustrate the time course and the Hct linearity obtained when the direct current voltage 1.0 V was applied to the sample in which the glucose value was 50 mg/dl. FIG. 12A and FIG. 12B illustrate the time course and the Hct linearity obtained when the direct current voltage 1.5 V was applied to the sample in which the glucose value was 50 mg/dl. FIG. 12C and FIG. 12D illustrate the time course and the Hct linearity obtained when the direct current voltage 2.0 V was applied to the sample in which the glucose value was 50 mg/dl. FIG. 12E and FIG. 12F illustrate the time course and the Hct linearity obtained when the direct current voltage 2.5 V was applied to the sample in which the glucose value was 50 mg/dl.

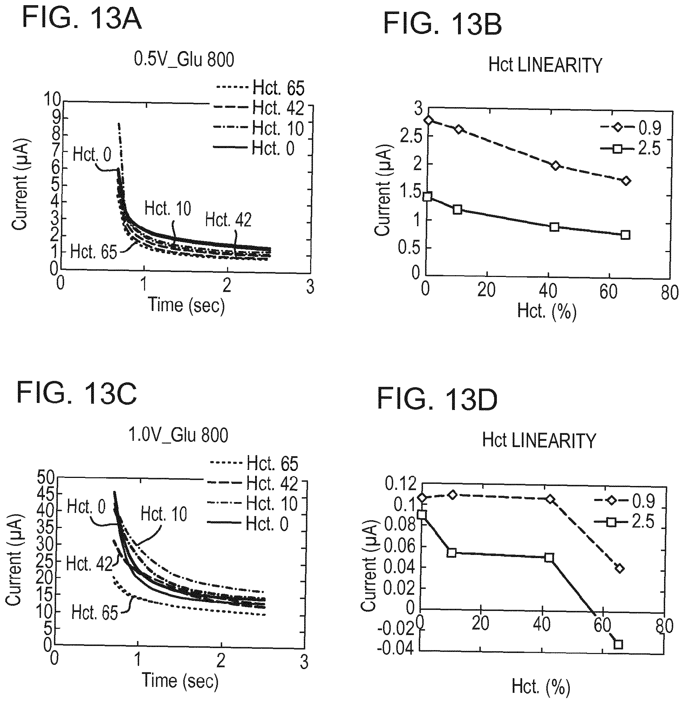

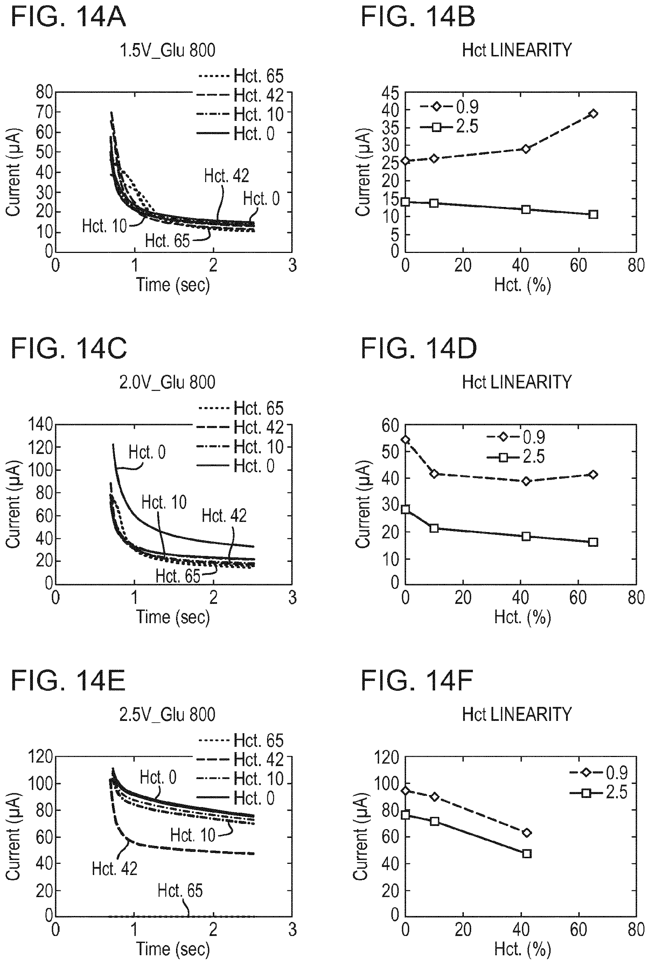

FIGS. 13A to 13D and FIGS. 14A to 14E illustrate the time courses and the Hct linearity provided when direct current voltages 0.5 V, 1.0 V, 1.5 V, 2.0 V, and 2.5 V were applied as the second signal to samples in which the glucose value was 800 mg/dl and the Hct values were 0%, 10%, 42%, and 65% respectively. Specifically, FIG. 13A and FIG. 13B illustrate the time course and the Hct linearity obtained when the direct current voltage 0.5 V was applied to the sample in which the glucose value was 800 mg/dl. FIG. 13C and FIG. 13D illustrate the time course and the Hct linearity obtained when the direct current voltage 1.0 V was applied to the sample in which the glucose value was 800 mg/dl. FIG. 14A and FIG. 14B illustrate the time course and the Hct linearity obtained when the direct current voltage 1.5 V was applied to the sample in which the glucose value was 800 mg/dl. FIG. 14C and FIG. 14D illustrate the time course and the Hct linearity obtained when the direct current voltage 2.0 V was applied to the sample in which the glucose value was 800 mg/dl. FIG. 14E and FIG. 14F illustrate the time course and the Hct linearity obtained when the direct current voltage 2.5 V was applied to the sample in which the glucose value was 800 mg/dl.

The following measurement was performed for both of the case in which the glucose value was 50 mg/dl and the case in which the glucose value was 800 mg/dl. That is, the application of the direct current voltage was started 0.5 second after the start of the measurement. The response current was measured at a point in time of 0.9 second (0.4 second after the start of the application) and a point in time of 2.5 seconds (2 seconds after the start of the application) after the start of the measurement. The Hct linearities illustrated in FIGS. 11B, 11D, 12B, 12D, 12F, 13B, 13D, 14B, 14D, 14F are based on the response currents obtained by the measurement as described above.

According to the results illustrated in FIGS. 11A to 11D and FIGS. 12A to 12F, when the applied the direct current voltage is 0.5 V, both of the glucose value and the Hct value cannot be measured, because the reagent is not provided on the counter electrode, and the voltage is insufficient. When the applied direct current voltage is 1.0 V and when the applied direct current voltage is 1.5 V, then it has been observed that the response current depends on the glucose value without depending on the Hct value. Further, when the applied direct current voltage is 2.0 V, the response current depends on the glucose value for a certain time after the start of the application. However, it has been observed that the response current slightly depends on the Hct value after the elapse of a given time (for example, 2 seconds). Then, when the applied direct current voltage is 2.5 V, the response current depends on the glucose value for a certain time after the start of the application. However, it has been observed that the response current depends on the Hct value after the elapse of a given time (for example, 2 seconds). Further, although not illustrated, when the applied direct current voltage is not less than 2.5V, the response current depends on the glucose value for a certain time after the start of the application, in the same manner as in the case of the direct current voltage 2.5V. However, it has been revealed that the response current depends on the Hct value after the elapse of a given time. However, bubbles are produced in the sample as the applied voltage is increased. Therefore, the upper limit of the applied voltage is about 7 V at which bubbles are not produced. Note that these results are obtained in the case of the ruthenium electrode. If any different electrode is used, the appropriate applied voltage is in a different range. Further, the appropriate applied voltage may be in a different range, depending on the type of salt.

Further, in the case of the results illustrated in FIGS. 13A to 13D and FIGS. 14A to 14F (glucose value was 800 mg/dl), the same or equivalent tendencies as those observed when the glucose value was 50 mg/dl were also observed. The structures and the arrangements explained in the embodiments can be appropriately combined with each other.

According to the embodiments, it is possible to accurately measure a target substance contained in a sample.

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to an illustration of the superiority and inferiority of the invention. Although the embodiments of the present invention have been described in detail, it should be understood that the various changes, substitutions, and alterations could be made hereto without departing from the scope of the invention, as defined by the claims.

## Claims

1. A method for measuring a target substance contained in a sample by using an analysis tool in which at least one working electrode provided with a reagent including an enzyme and at least one counter electrode are arranged in a flow passage, the measuring method comprising:
applying a first signal, which is a direct current signal, having a first value to the sample disposed in the flow passage by using the working electrode and the counter electrode;
measuring a first electric response value of the sample to the first signal;
applying continuously for a given time a second signal, which is a direct current signal, having a second value higher than the first value to the sample disposed in the flow passage by using the working electrode and the counter electrode;
measuring a second electric response value of the sample within the given time to the second signal, the second electric response value indicating amount of electric charge generated by electrolysis of water in the sample; and
correcting a value obtained from the first electric response value based on the second electric response value.

2. The method according to claim 1, wherein the counter electrode, which is included in the at least one counter electrode and which is not provided with any salt component, is used to apply the second signal.

3. The method according to claim 1, wherein the counter electrode, which is included in the at least one counter electrode and which is not provided with any mediator, is used to apply the second signal.

4. The method according to any of claims 1 to 3, wherein the working electrode, which is used to apply the first signal, is used to apply the second signal.

5. The method according to claim 1 or 4, wherein when the analysis tool includes, as the at least one counter electrode, a first counter electrode which is provided with the reagent including the enzyme and a mediator and a second counter electrode which is not provided with the reagent including the enzyme and the mediator, then the first counter electrode is used to apply the first signal, and the second counter electrode is used to apply the second signal.

6. The method according to any of claims 1 to 3, wherein the counter electrode, which is used to apply the first signal, is used to apply the second signal.

7. The method according to any of claims 4 to 6, wherein the counter electrode, which is included in the at least one counter electrode and which is not provided with the reagent including the enzyme and a mediator, is used to apply the first signal and apply the second signal.

8. The method according to any preceding claim, wherein the sample is blood.

9. The method according to any preceding claim, wherein the target substance is glucose.

10. The method according to any preceding claim, wherein the second electric response value of the sample is a value which indicates hematocrit.

11. The method according to claim 10, wherein the first electric response value of the sample is a value which indicates glucose value before being corrected by the value which indicates hematocrit.

12. The measuring method according to any preceding claim, wherein the second signal is a direct current voltage and the second value ranges from 1V to 7V.

13. A measuring apparatus for measuring a target substance contained in a sample by using an analysis tool in which at least one working electrode provided with a reagent including an enzyme and at least one counter electrode are arranged in a flow passage, the measuring apparatus comprising:
a first measuring unit configured to apply a first signal, which is a direct current signal, having a first value to the sample disposed in the flow passage by using the working electrode and the counter electrode, and configured to measure a first electric response value of the sample to the first signal;
a second measuring unit configured to apply continuously for a given time a second signal, which is a direct current signal, having a second value higher than the first value to the sample disposed in the flow passage by using the working electrode and the counter electrode, and configured to measure a second electric response value of the sample within the given time to the second signal, the second electric response value indicating amount of electric charge generated by electrolysis of water in the sample; and
a correcting unit configured to correct a value obtained from the first electric response value based on the second electric response value.
